(19) Europäisches Patentamt
European Patent Office
Office européen des brevets

(11) Veröffentlichungsnummer : **0 039 835**
**B1**

# (12) EUROPÄISCHE PATENTSCHRIFT

(45) Veröffentlichungstag der Patentschrift : **09.03.83**

(51) Int. Cl.³ : **C 07 D277/82**

(21) Anmeldenummer : **81103225.9**

(22) Anmeldetag : **29.04.81**

## (54) Verfahren zur Herstellung von 2-Amino-6-nitrobenzthiazol.

(30) Priorität : **10.05.80 DE 3018028**

(43) Veröffentlichungstag der Anmeldung : **18.11.81 Patentblatt 81/46**

(45) Bekanntmachung des Hinweises auf die Patenterteilung : **09.03.83 Patentblatt 83/10**

(84) Benannte Vertragsstaaten : **BE CH DE FR GB IT LI**

(56) Entgegenhaltungen : **DE A 2 601 700**

(73) Patentinhaber : **BAYER AG**
**Zentralbereich Patente, Marken und Lizenzen**
**D-5090 Leverkusen 1, Bayerwerk (DE)**

(72) Erfinder : **Horstmann, Walter, Dr.**
**Eichenweg 17**
**D-5060 Bergisch Gladbach 2 (DE)**
Erfinder : **Sommer, Richard, Dr.**
**105 Pariot Place**
**Summerville South Carolina 29483 (US)**
Erfinder : **Trautwein, Hans, Dr.**
**In der Follmühle 24**
**D-5068 Odenthal-Voiswinkel (DE)**
Erfinder : **Wolfrum, Gerhard, Dr.**
**Domblick 17**
**D-5090 Leverkusen 3 (DE)**

**EP 0 039 835 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von 2-Amino-6-nitrobenzthiazol

Die vorliegende Erfindung betrifft ein Verfahren zur Herstellung von 2-Amino-6-nitrobenzthiazol.

Es ist bereits bekannt, die Titelverbindung durch direkte Nitrierung von 2-Aminobenzthiazol herzustellen (vgl. J. Chem. Soc. *1930*, 2203). Dieses Verfahren weist jedoch den Nachteil auf, daß es ein Isomerengemisch liefert, in dem die erwünschte 6-Nitroverbindung nur zu ca. 20 % enthalten ist.

Es wurde nun überraschenderweise gefunden, daß man die 6-Nitroverbindung in hoher Selektivität erhält, wenn man nicht das 2-Aminobenzthiazol als solches, sondern dessen Acylierungsprodukte nitriert und die Acylgruppen anschließend abspaltet.

Geeignete 2-Acylaminobenzthiazole sind insbesondere solche der Formel

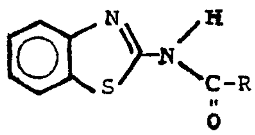

(I)

in welcher R Wasserstoff, Alkyl, Alkoxy, Trifluormethyl oder Carboxyalkyl bedeuten. Als Alkyl-, Alkoxy- bzw. Carboxyalkylreste kommen insbesondere solche mit 1 bis 4 Kohlenstoffatomen in Betracht. Bevorzugt sind der Formyl- und der Acetylrest.

Die als Ausgangsmaterial verwendeten 2-Acylaminobenzthiazole sind bekannt bzw. nach bekannten Verfahren durch Umsetzung von 2-Aminobenzthiazol mit entsprechenden Säurechloriden oder -anhydriden, Chlorkohlensäureestern bzw. Oxalsäureestern leicht erhältlich (vgl. Annalen der Chemie *212*, 326 (1882) ; US-PS 2 833 689 ; DOS 2 656 468)

2-Aminobenzthiazol gewinnt man wiederum am einfachsten durch Einwirkung von Hydroxylamin auf Benzthiazol (vgl. Annalen der Chemie *419*, 65 (1919)), welches ein Abfallprodukt bei der großtechnischen Herstellung von 2-Mercaptobenzthiazol ist.

In der Verwendung dieses unerwünschten Nebenproduktes, für welches es bislang keine befriedigenden Verwertungsmöglichkeiten gab, zur Herstellung von 2-Amino-6-nitrobenzthiazol ist ein weiterer wichtiger Vorteil des erfindungsgemäßen Verfahrens zu sehen.

Die Nitrierung der Acylverbindungen erfolgt nach an sich bekannten Methoden. Zweckmäßigerweise geht man dabei so vor, daß man die Acylverbindung in der 2- bis 6-fachen Gewichtsmenge Schwefelsäure löst und dann mit einer Mischung von Salpetersäure und Schwefelsäure — vorzugsweise bei 0 bis 50 °C — nitriert. Der Salpetersäureanteil in der Mischsäure soll dabei 5 bis 60 Gew.-% und das Molverhältnis von Acylaminobenzthiazol zur Salpetersäure 1,0 : 1,0 bis 1,5 betragen.

Die Nitrierung der 2-Acylaminobenzthiazole kann auch mit Salpetersäure allein erfolgen, wobei das Gewichtsverhältnis der Komponenten 1 : 2 bis 1 : 10 betragen kann.

Die Aufarbeitung des Nitriergemisches erfolgt in üblicher Weise, z. B. durch Austragen auf Eis.

Auch die Abspaltung der Acylgruppe erfolgt in an sich bekannter Weise durch Verseifung mit verdünnten Mineralsäuren, Alkalilaugen oder Ammoniaklösung in wäßriger oder wäßrig-alkoholischer Lösung bzw. Suspension bei erhöhten Temperaturen und gegebenenfalls unter Druckanwendung, wobei als Alkoholkomponente in der wäßrig-alkoholischen Lösung Methanol, Ethanol oder Isopropanol eingesetzt werden können.

Als vorteilhaft haben sich Temperaturen von 50 bis 150 °C, vorzugsweise von 60 bis 100 °C und Drucke bis 50 bar erwiesen.

Je nach Wahl der Versuchsbedingungen fällt 2-Amino-6-nitrobenzothiazol in zwei Kristallmodifikationen an. Die mit A bezeichnete Modifikation besteht aus nadelförmigen, hellgelben Kristallen von geringem Schüttgewicht, die Modifikation B aus derben, gelbbraun gefärbten Kristallen von relativ hoher Dichte. Beide Modifikationen unterscheiden sich röntgenographisch durch ihre Debye-Scherrer-Diagramme :

Modifikation A

| α-Werte | Intensität (geschätzt) |
|---|---|
| 3,20 Å | 100 |
| 6,13 Å | 40 |
| 7,94 Å | 40 |
| 3,75 Å | 20 |

Modifikation B

| α-Werte | Intensität (geschätzt) |
|---|---|
| 3,53 Å | 100 |
| 5,22 Å | 60 |
| 3,05 Å | 40 |
| 4,67 Å | 20 |

2-Amino-6-nitrobenzothiazol stellt ein wichtiges Zwischenprodukt zur Herstellung von Azofarbstoffen dar (vgl. Jap. Patentpublikation 13 389/69).

Das nach dem erfindungsgemäßen Verfahren gewonnene 2-Amino-6-nitrobenzothiazol kann ohne weitere Reinigung zur Herstellung von Farbstoffen herangezogen werden.

## Beispiel 1

192 g (1,0 Mol) 2-Acetylaminobenzthiazol (Annalen *212*, 326 (1882)) werden bei 20 bis 30 °C in 490 g Schwefelsäuremonohydrat eingetragen. Man kühlt auf 5 bis 10 °C ab und nitriert in diesem Temperaturintervall mit 200 g einer Mischsäure, die 31,5 % Salpetersäure enthält. Nach Eintropfen der Mischsäure rührt man 2 Stunden bei 10 bis 15 °C nach und trägt dann auf 1 000 g Eis aus. Der Niederschlag wird isoliert und mit ca. 5 l Wasser portionsweise gewaschen. Man erhält 1 100 g wasserfeuchtes 2-Acetylamino-6-nitrobenzthiazol.

Den feuchten Preßkuchen suspendiert man in 1 650 ml Methanol. Man erhitzt auf 60 °C und stellt mit konzentrierter Natronlauge einen pH-Wert von 10,5 ein. Im Verlaufe von 5 Stunden werden, um diesen pH-Wert zu halten, ca. 60 ml Natronlauge verbraucht. Nun kühlt man auf 20 °C ab, isoliert das auskristallisierte 2-Amino-6-nitrobenzthiazol (Modifikation B), wäscht mit 200 ml Methanol und anschließend mit Wasser alkalifrei. Nach dem Trocknen bei 50 °C im Vakuumschrank erhält man 171 g Ausbeute, Schmelzpunkt 248 bis 252 °C. Der Gehalt an 2-Amino-5-nitrobenzothiazol liegt bei 0,3 %. Diese Qualität von 2-Amino-6-nitrobenzthiazol kann ohne weitere Reinigungsoperationen direkt zur Farbstoffherstellung verwendet werden.

## Beispiel 2 (Vergleichsbeispiel)

Analog der Vorschrift von Hunter und Jones (J.Chem.Soc. *1930*, 2203) werden in 90 ml Salpetersäure der Dichte 1,5 bei Temperaturen zwischen 0 und 5 °C 15,0 g (0,1 Mol) 2-Amino-benzthiazol im Verlaufe von 15 Minuten eingetragen. Man rührt eine Stunde lang nach, trägt das Reaktionsgemisch auf 900 ml Eiswasser aus und stellt mit ca. 170 ml 25 %iger Ammoniaklösung auf einen pH-Wert von 8 ein. Der Niederschlag wird isoliert, mit Wasser neutral gewaschen und im Vakuum bei 50 °C getrocknet. Man erhält 19,2 g eines Produkts, das zu 70-80 % aus 2-Amino-5-nitro-benzthiazol, zu 15-20 % aus 2-Amino-6-nitro-benzthiazol und zu 5-10% aus weiteren Mono- und Dinitro-Isomeren besteht. Zur technischen Herstellung von 2-Amino-6-nitro-benzthiazol ist diese Methode ungeeignet.

## Beispiel 3

96 g (0,5 Mol) 2-Acetylaminobenzthiazol trägt man bei 0 bis 5 °C in 300 g 94 %ige Salpetersäure ein. Anschließend rührt man ohne Kühlung von außen 3 Stunden lang nach. Dabei steigt die Temperatur bis 28 °C an. Man trägt den Ansatz auf 600 g Eis-Wasser-Gemisch aus, saugt ab und wäscht mit 2 l Wasser anteilweise nach. Man erhält 459 g Paste von 2-Acetylamino-6-nitrobenzthiazol. Diese suspendiert man in 900 ml Methanol und verseift wie im Beispiel 1 beschrieben. Die Ausbeute beträgt 67 g 2-Amino-6-nitrobenzthiazol vom Schmelzpunkt 246 bis 249 °C. Der Gehalt an 2-Amino-5-nitrobenzthiazol liegt unter 0,1 %. Das Produkt kann ohne weitere Reinigung zur Farbstoffherstellung eingesetzt werden.

## Beispiel 4

19 g (0,09 Mol) Benzthiazolyl-(2)-carbaminsäureethylester werden, wie im Beispiel 1 beschrieben, nitriert. Die erhaltene wasserfeuchte Paste von 6-Nitro-benzthiazolyl-(2)-carbaminsäureethylester wird, wie im Beispiel 1 beschrieben, bei pH 10 bis 12 verseift und aufgearbeitet. Man erhält 9,8 g 2-Amino-6-nitrobenzthiazol vom Schmelzpunkt 249 bis 252 °C. Der Gehalt an isomeren Nitroverbindungen liegt unter 1 %.

## Beispiel 5

35,6 g 2-Benzthiazolyl-formamid (hergestellt nach Angaben von C.W. Huffmann, J. Org. Chem. *23*, 727-729 [1958]) werden, wie im Beispiel 1 für 2-Acetylaminobenzthiazol beschrieben, nitriert und aufgearbeitet. Man erhält 177 g wasserfeuchte Paste, entsprechend 35,7 g 6-Nitro-benzthiazolyl-(2)-formamid vom Schmelzpunkt 262-266 °C. Nach Umlösen aus viel Methylglykolacetat schmilzt das Produkt bei 268-269 °C. Die wasserfeuchte Paste wird in 350 ml Methanol suspendiert und wie im Beispiel 1 verseift. Nach dem Trocknen erhält man 30,6 g 6-Nitro-2-aminobenzthiazol, Schmelzpunkt 248-251 °C. Der Gehalt an dem 5-Isomeren liegt 0,05 %, der des 4- und 7-Isomeren 1,2 %. Das Rohprodukt kann ohne weitere Reinigung zur Farbstoffherstellung verwendet werden.

## Beispiel 6

178 g (1,0 Mol) 2-Benzthiazolyl-formamid werden, wie im Beispiel 1 für 2-Acetylaminobenzthiazol

beschreiben, nitriert. Dann trägt man die Nitriermasse auf 1 450 g Eiswasser aus, so daß die resultierende Schwefelsäurekonzentration ca. 30 % beträgt. Man erwärmt die Suspension von 2-(6-Nitrobenzthiazolyl)-formamid auf 60 °C und rührt 2 Stunden lang bei dieser Temperatur nach. Nachdem man auf 30 °C abgekühlt hat, stumpft man das Reaktionsgemisch durch Zugabe von 1 420 ml 25 %iger Natronlauge bis zum pH-Wert 5-6 ab, filtriert 2-Amino-6-nitrobenzthiazol ab und wäscht mit 3 Litern Wasser anteilweise nach. Nach dem Trocknen bei 80 °C im Vakuumschrank erhält man 194 g Ausbeute vom Schmelzpunkt 248-250 °C. Das auf diese Weise gewonnene 2-Amino-6-nitrobenzthiazol enthält maximal je 1,5 % der isomeren 4-, 5- und 7-Nitroverbindungen und läßt sich ohne besondere Reinigung zur Herstellung von Farbstoffen einsetzen.

**Ansprüche**

1. Verfahren zur Herstellung von 2-Amino-6-nitrobenzthiazol, dadurch gekennzeichnet, daß man ein 2-Acylaminobenzthiazol nitriert und das dabei erhaltene 6-Nitro-2-acylaminobenzthiazol verseift.

2. Verfahren nach Anspruch 1, dadurch gekennzeichnet, daß man als 2-Acylaminobenzthiazole 2-Formyl- und 1-Acetylaminobenzthiazol einsetzt.

3. Verfahren nach Anspruch 1, dadurch gekennzeichnet, daß man die Nitrierung bei 0 bis 50 °C durchführt.

4. Verfahren nach Anspruch 1 und 3, dadurch gekennzeichnet, daß man die Nitrierung bei 0 bis 10 °C durchführt.

5. Verfahren nach Anspruch 1, dadurch gekennzeichnet, daß man zur Nitrierung Mischsäuren verwendet, die 5 bis 60 Gew.-% Salpetersäure enthalten, wobei das Molverhältnis von Acylaminobenzthiazol zur Salpetersäure 1,0 : 1,0 bis 1,5 beträgt.

6. Verfahren nach Anspruch 1, dadurch gekennzeichnet, daß man die Verseifung der 6-Nitro-2-acylaminobenzthiazole mit Alkalihydroxiden, Ammoniak oder Mineralsäuren in wäßriger oder wäßrig-alkoholischer Lösung bzw. Suspension bei Temperaturen von 20 bis 100 °C durchführt.

7. Verfahren nach Anspruch 6, dadurch gekennzeichnet, daß man als Alkoholkomponente in der wäßrig-alkoholischen Lösung Methanol, Ethanol oder Isopropanol verwendet.

**Claims**

1. Process for the preparation of 2-amino-6-nitrobenzothiazole, characterised in that a 2-acylaminobenzothiazole is nitrated and the 6-nitro-2-acylaminobenzothiazole thereby obtained is saponified.

2. Process according to Claim 1, characterised in that 2-formyl- and 1-acetyl-aminobenzothiazole are employed as the 2-acylaminobenzothiazoles.

3. Process according to Claim 1, characterised in that the nitration is carried out at 0 to 50 °C.

4. Process according to Claim 1 and 3, characterised in that the nitration is carried out at 0 to 10 °C.

5. Process according to Claim 1, characterised in that mixed acids which contain 5 to 60 % by weight of nitric acid are used for the nitration, the molar ratio of acylaminobenzothiazole to nitric acid being 1.0 : 1.0 to 1.5.

6. Process according to Claim 1, characterised in that the saponification of the 6-nitro-2-acylaminobenzothiazoles is carried out with alkali metal hydroxides, ammonia or mineral acids in aqueous or aqueous-alcoholic solution or suspension at temperatures from 20 to 100 °C.

7. Process according to Claim 6, characterised in that methanol, ethanol or isopropanol is used as the alcohol component in the aqueous-alcoholic solution.

**Revendications**

1. Procédé de préparation du 2-amino-6-nitrobenzothiazole caractérisé en ce que l'on nitre un 2-acylaminobenzothiazole et on saponifie le 6-nitro-2-acylaminobenzothiazole obtenu.

2. Procédé selon la revendication 1, caractérisé en ce que l'on utilise en tant que 2-acylaminobenzothiazoles le 2-formyl et le 1-acétylaminobenzothiazole.

3. Procédé selon la revendication 1, caractérisé en ce que la nitration est effectuée à une température de 0 à 50 °C.

4. Procédé selon les revendications 1 et 3, caractérisé en ce que la nitration est effectuée à une température de 0 à 10 °C.

5. Procédé selon la revendication 1 caractérisé en ce que, pour la nitration, on utilise des mélanges sulfonitriques qui contiennent de 5 à 60 % en poids d'acide nitrique, avec un rapport molaire acylaminobenzothiazole/acide nitrique de 1,0 : 1,0 à 1,5.

6. Procédé selon la revendication 1, caractérisé en ce que l'on saponifie le 6-nitro-2-acylaminobenzothiazole à l'aide d'hydroxydes alcalins, d'ammoniac ou d'acides minéraux en solution ou suspension aqueuse ou hydroalcoolique à des températures de 20 à 100 °C.

7. Procédé selon la revendication 6, caractérisé en ce que l'on utilise en tant que composant alcool de la solution hydroalcoolique du méthanol, de l'éthanol ou de l'isopropanol.